(19) Europäisches Patentamt
European Patent Office
Office européen des brevets

(11) EP 4 302 781 A1

(12) **EUROPEAN PATENT APPLICATION**
published in accordance with Art. 153(4) EPC

(43) Date of publication:
**10.01.2024 Bulletin 2024/02**

(21) Application number: **22763395.5**

(22) Date of filing: **03.03.2022**

(51) International Patent Classification (IPC):
***A61K 47/24*** (2006.01) ***A61K 8/11*** (2006.01)
***A61K 9/127*** (2006.01) ***A61K 47/14*** (2017.01)
***A61P 17/00*** (2006.01) ***A61Q 19/00*** (2006.01)

(52) Cooperative Patent Classification (CPC):
**A61K 8/11; A61K 9/127; A61K 47/14; A61K 47/24; A61P 17/00; A61Q 19/00**

(86) International application number:
**PCT/JP2022/009187**

(87) International publication number:
**WO 2022/186343 (09.09.2022 Gazette 2022/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.03.2021 JP 2021033431**

(71) Applicants:
- **TOYO BEAUTY CO., LTD.**
**Osaka-shi, Osaka 537-0021 (JP)**
- **Dainihon Kasei Co., Ltd.**
**Osaka-shi, Osaka, 541-0045, (JP)**

(72) Inventors:
- **HISAMA Masayoshi**
**Osaka-shi, Osaka 537-0021 (JP)**
- **HISAMA Sanae**
**Osaka-shi, Osaka 537-0021 (JP)**
- **IKEDA Motoyasu**
**Osaka-shi, Osaka 537-0021 (JP)**
- **YOSHIO Kimio**
**Osaka-shi, Osaka 537-0021 (JP)**
- **AONO Miki**
**Osaka-shi, Osaka 541-0045 (JP)**

(74) Representative: **Grünecker Patent- und Rechtsanwälte PartG mbB**
**Leopoldstraße 4**
**80802 München (DE)**

# (54) TOPICAL MICROPARTICLE CAPSULE PREPARATION AND DERMATOLOGICAL TOPICAL AGENT

(57) Provided is a topical fine-particulate capsule preparation including a fine-particulate capsule preparation in which an amphiphilic capsule membrane of emulsified particles encapsulates an active ingredient. The amphiphilic capsule membrane is composed of an amphiphilic ingredient containing isostearyl ascorbyl phosphate or a metal salt thereof represented by the following Chemical Formula, which can be decomposed by phosphatase, which is an enzyme widely distributed in the skin and the like. In the following Chemical Formula, M is a hydrogen atom or a monovalent metal ion.

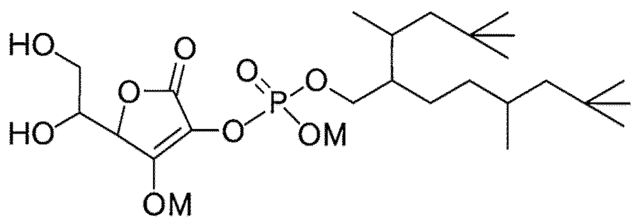

EP 4 302 781 A1

FIG. 1

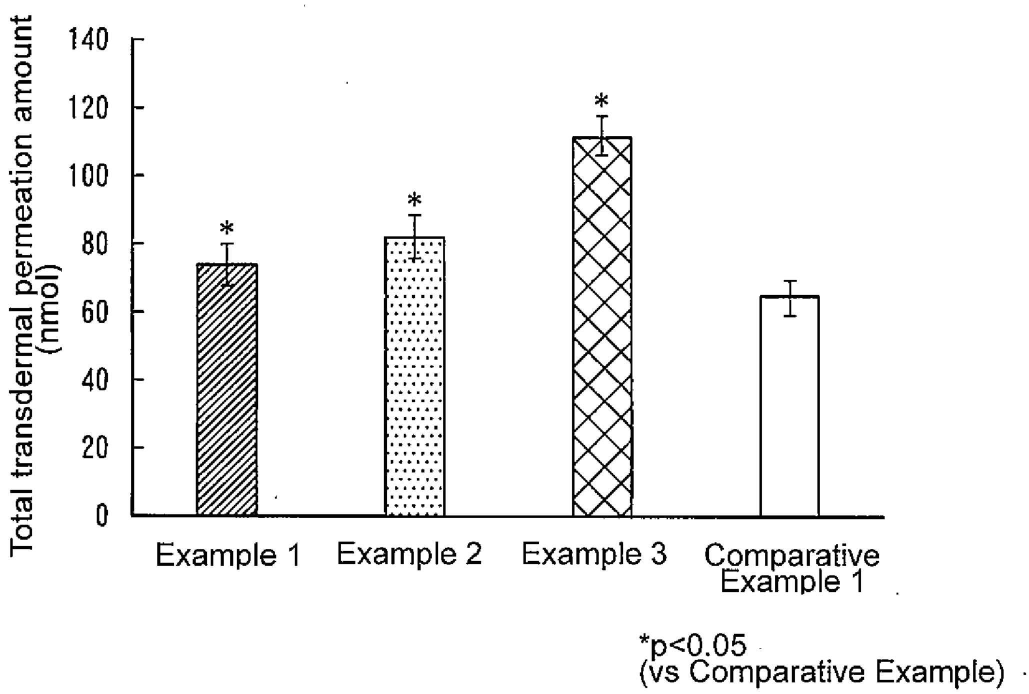
Total transdermal permeation amount (nmol)
140
120
100
80
60
40
20
0
*
*
*
Example 1
Example 2
Example 3
Comparative Example 1
*p<0.05
(vs Comparative Example)

## Description

### TECHNICAL FIELD

The present invention relates to a topical fine-particulate capsule preparation having skin permeability and capable of releasing an encapsulated active ingredient into the skin; and a skin topical agent including the same.

### BACKGROUND ART

Skin is composed of epidermis, dermis, and subcutaneous tissue. The outermost horny layer of the epidermis includes multilayer membranes of flat dead cells composed of a keratin matrix, which have a so-called "block mortar" structure. The multilayer membranes are hierarchical and regularly arranged like bricks of a brick wall, among which an intercellular lipid is filled.

With such a structure, the skin has a barrier function of preventing invasion of various allergens and bacteria from the outside, and also controlling evaporation of moisture from the inside of the skin.

However, due to such a barrier function, it is difficult to allow a physiologically active ingredient or the like to permeate the skin from the outside of the skin to the inside. Accordingly, various transdermal absorption promoting systems have been studied.

For example, in skin topical agents such as pharmaceuticals and cosmetic products, pulverization of pigments and powders by application of nanotechnology, liquid crystal emulsification, capsule preparations including liposomes, and the like are known.

Phospholipids, which are major components of liposomes, are amphipathic substances having a hydrophilic phosphate moiety and a hydrophobic fatty acid ester moiety. It is well known by the Bangham method (1964) that phospholipids form a closed vesicle (liposome) in an aqueous solution, and it is known that the liposome can be applied as a drug delivery system.

That is, the liposome is composed of a single layer or a plurality of layers of lipid bilayer membranes, and when water is used as a dispersion medium, the hydrophobic part gathers inside, and the hydrophilic part faces outside, so that the liposome has a spherical capsule structure. Accordingly, a water-soluble medicinal ingredient can be confined in the hydrophilic part, and an oil-soluble medicinal ingredient can be confined in the hydrophobic part.

Fine-particulate capsule preparations including such liposomes have attracted attention mainly in the field of medicine for application to drug delivery systems. Also, in the field of cosmetic products, they can release an active ingredient and allow the active ingredient effectively to permeate the skin in response to a change in environment when applied to the skin.

For example, there is known a drug release system that releases an encapsulated ingredient through membrane disintegration using heat, temperature, or pH as a controlling factor, such as a temperature sensitive liposome (below-identified Patent Document 1), a pH responsive liposome (below-identified Patent Document 2), and a temperature and pH responsive liposome (below-identified Patent Document 3).

In addition, vitamin C is known as a physiologically active ingredient for skin, but is so water-soluble that it hardly permeates the skin. Accordingly, in order to enhance the permeability, a derivative is well known in which a lipophilic isostearic acid ester is bonded (below-identified Non-Patent Document 1). This vitamin C derivative is converted into vitamin C by phosphatase abundantly present in the skin, which vitamin C can be expected to exert a protective action against active oxygen due to antioxidant properties, a whitening action, and the like.

### PRIOR ART DOCUMENTS

### PATENT DOCUMENTS

Patent Document 1: Japanese Unexamined Patent Application Publication No. 2006-306794
Patent Document 2: Japanese Unexamined Patent Application Publication No. 2010-209012
Patent Document 3: Japanese Unexamined Patent Application Publication No. 2015-209494

### NON-PATENT DOCUMENT

Non-Patent Document 1: Skin Pharm. Physol., 21, 235-248 (2008)

## SUMMARY OF THE INVENTION

### PROBLEMS TO BE SOLVED BY THE INVENTION

However, in a drug release system using a fine-particulate capsule such as a liposome, a control technique for sufficiently efficiently delivering an ingredient encapsulated in the liposome into a living body towards a target site such as constituent cells at a disease site and an aging site in the skin has not been sufficiently established.

For example, various topical environmental factors act on the skin in the living environment of human, and the temperature, pH, and the like on the skin surface may unexpectedly greatly fluctuate due to the topical environmental factors, so that an encapsulated ingredient may not be reliably transported into a living body only by the environmental responsiveness of a fine-particulate capsule preparation.

Accordingly, in order to allow an encapsulated ingredient to efficiently act on the target cells in a living body via the skin while responding to various topical environments, there is a need for a technique of not only enhancing the permeability to the skin and the retention in a living body, but also making the encapsulated active ingredient reach the target site or the target cells in the skin, and then reliably releasing the encapsulated active ingredient at an optimal timing.

An object of the present invention is to solve the problems as described above, and to create a topical fine-particulate capsule preparation which can have stable permeability to the skin as a transdermal absorptiontype preparation or a skin topical agent, directly and efficiently transport an encapsulated ingredient to the inside of the skin to which the preparation has permeated, and reliably release the encapsulated active ingredient in the skin.

### MEANS FOR SOLVING THE PROBLEMS

The inventors of the present application have researched and developed a fine-particulate capsule preparation that has skin permeability, and that can release an encapsulated ingredient when the capsule is broken by an enzyme that is resident in the skin; and have found that the release in the skin can be controlled by the composition of the preparation. Furthermore, the inventors have found an effective delivery action of the encapsulated ingredient into the skin also in a skin topical agent containing the fine-particulate capsule preparation of the present invention, leading to the accomplishment of the present invention.

That is, in order to solve the above problems, the present invention provides a topical fine-particulate capsule preparation comprising a fine-particulate capsule preparation in which an amphiphilic capsule membrane of emulsified particles encapsulates an active ingredient, wherein the amphiphilic capsule membrane is composed of an amphiphilic ingredient containing isostearyl ascorbyl phosphate or a metal salt thereof represented by the following Chemical Formula 1.

[Chemical Formula 1]

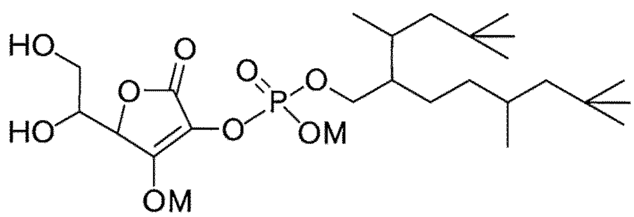

(M in Chemical Formula 1 is a hydrogen atom or a monovalent metal ion.)

The topical fine-particulate capsule preparation of the present invention, which is composed as described above, has moderate lipid solubility so as to be easily incorporated into the skin, because the isostearyl ascorbyl phosphate or the metal salt thereof (isostearyl ascorbyl phosphate dimetal salt) contained in the amphipathic capsule membrane is L-ascorbic acid 2-phosphate having an alkyl group in which a phosphate moiety is branched, thereby efficiently transporting the encapsulated active ingredient to the inside of the skin to which the preparation has permeated.

Then, because the isostearyl ascorbyl phosphate or the metal salt thereof is rapidly decomposed into L-ascorbic acid, phospholipids and the like by phosphatase, which is an enzyme widely distributed in the skin to which the preparation has permeated and the like, the amphiphilic capsule membrane is broken in a living body. Accordingly, the active ingredient encapsulated in the emulsified particles can be reliably released. In this way, a topical fine-particulate capsule preparation having excellent transdermal transportation properties into the skin is obtained.

In order to enhance the skin permeability, the amphiphilic capsule membrane preferably has hydrophilicity and

lipophilicity corresponding to the lipid solubility and hydrophilicity inherent in the skin, and preferably contains a phospholipid and a ceramide analog which are ingredients composing the skin. The ceramide analog preferably comprises one or more ceramide analogs selected from a natural ceramide, a glycosylated ceramide compound, a synthetic pseudo ceramide, sphingosine, and phytosphingosine.

In addition, a polyglycerin fatty acid ester that can be synthesized and can cope with a wide range from hydrophilicity to lipophilicity is applicable as a component of the amphiphilic capsule membrane, because the polyglycerin fatty acid ester can also have acid resistance, base resistance, and heat resistance.

The emulsified particles of the fine-particulate capsule preparation composed as described above encapsulating the active ingredient with the amphipathic capsule membrane are micrometer-sized or nanometer-sized capsule fine particles so as to enhance the permeability to the skin and efficiently transport the medicinal ingredient. For example, the emulsified particles preferably have a particle diameter of 100 to 300 nm.

In addition, the topical fine-particulate capsule preparation having the above composition in which the active ingredient is an active ingredient for skin physiological activity is applicable to a skin topical agent such as cosmetics containing a physiologically active ingredient as an active ingredient.

## EFFECTS OF THE INVENTION

Since the capsule preparation of the present invention is a fine-particulate capsule preparation containing isostearyl ascorbyl phosphate or a metal salt thereof represented by Chemical Formula 1 above in an amphiphilic capsule membrane, there is an advantage that the capsule preparation is a topical fine-particulate capsule preparation which has sufficient permeability to the skin as a skin topical agent, which can efficiently transport an encapsulated ingredient into the skin, and which can reliably release the encapsulated ingredient into the skin without being affected by environmental factors outside the skin.

In addition, a skin topical agent containing the topical fine-particulate capsule preparation which can produce the above effect has an advantage that the skin physiological activity can be sufficiently enhanced by the active ingredient.

## BRIEF DESCRIPTION OF THE DRAWINGS

Fig. 1 is a graph showing the total transdermal permeation amounts according to Examples 1 to 3 and Comparative Example 1 that were confirmed in transdermal absorbability tests.

Fig. 2 is a graph showing the total transdermal permeation amounts according to Examples 4 to 6 and Comparative Example 2 that were confirmed in transdermal absorbability tests.

Fig. 3 is a graph showing the total transdermal permeation amounts according to Examples 7 to 9 and Comparative Example 3 that were confirmed in transdermal absorbability tests.

Fig. 4 is a graph showing the total transdermal permeation amounts according to Examples 10 to 12 and Comparative Example 4 that were confirmed in transdermal absorbability tests.

Fig. 5 is a graph showing the total transdermal permeation amounts according to Examples 13 to 15 and Comparative Example 5 that were confirmed in transdermal absorbability tests.

Fig. 6 is a graph showing the total transdermal permeation amounts according to Examples 16 to 18 and Comparative Example 6 that were confirmed in transdermal absorbability tests.

Fig. 7 is a graph showing the delivery rates of encapsulated ingredients according to Examples 1 to 3 and Comparative Example 1 in the skin that were confirmed in the transdermal absorbability tests.

Fig. 8 is a graph showing the delivery rates of encapsulated ingredients according to Examples 4 to 6 and Comparative Example 2 in the skin that were confirmed in the transdermal absorbability tests.

Fig. 9 is a graph showing the delivery rates of encapsulated ingredients according to Examples 7 to 9 and Comparative Example 3 in the skin that were confirmed in the transdermal absorbability tests.

Fig. 10 is a graph showing the delivery rates of encapsulated ingredients according to Examples 10 to 12 and Comparative Example 4 in the skin that were confirmed in the transdermal absorbability tests.

Fig. 11 is a graph showing the delivery rates of encapsulated ingredients according to Examples 13 to 15 and Comparative Example 5 in the skin that were confirmed in the transdermal absorbability tests.

Fig. 12 is a graph showing the delivery rates of encapsulated ingredients according to Examples 16 to 18 and Comparative Example 6 in the skin that were confirmed in the transdermal absorbability tests.

## BEST MODE FOR CARRYING OUT THE INVENTION

A topical fine-particulate capsule preparation according to an embodiment of the present invention that reacts

with an enzyme and encapsulates an active ingredient with an amphiphilic capsule membrane comprises, as a component of the amphiphilic capsule membrane, a vitamin C derivative comprising isostearyl ascorbyl phosphate or a metal salt thereof represented by the above Chemical Formula 1; and, as another component, emulsified particles employing at least one selected from a phospholipid, a ceramide, and a polyglycerin fatty acid ester.

The emulsified particles are present, for example, as an emulsion composition, while being dispersed as fine particles in a mixture including a lipophilic active ingredient serving as a dispersoid, water or a hydrophilic ingredient serving as a dispersion medium, and an amphipathic substance such as a surfactant, an emulsifier, an emulsion aid, or an emulsion stabilizer.

That is, the topical fine-particulate capsule preparation is a generic term including a dosage form of a fine-particulate capsule preparation having a micrometer size to a nanometer size, and obtained by a well-known dispersion and emulsification step of mixing an emulsion composition using a homogenizer or a predetermined high-speed mixer to prepare fine emulsified particles in a uniform dispersion state; and, if necessary, a mechanical emulsification step.

In addition, it is apparent from test results described later that especially by using a dosage form of a nanometer-sized fine-particulate capsule preparation, the effect is more excellent

The isostearyl ascorbyl phosphate or its metal salt (isostearyl ascorbyl phosphate dimetal salt) used in the present invention is L-ascorbic acid 2-phosphate having an alkyl group in which a phosphate moiety is branched, and is a well-known compound having excellent transdermal absorbability into the skin and metabolic properties, because the L-ascorbic acid 2-phosphate has moderate lipid solubility and is easily incorporated into the skin, and then rapidly decomposed into L-ascorbic acid, a phospholipid, and the like by phosphatase, which is an enzyme widely distributed in a living body.

Specific examples of the isostearyl ascorbyl phosphate dimetal salt include a disodium isostearyl ascorbyl phosphate represented by the following Chemical Formula 2, and, as another alkali metal salt, e.g., a potassium salt.

[Chemical Formula 2]

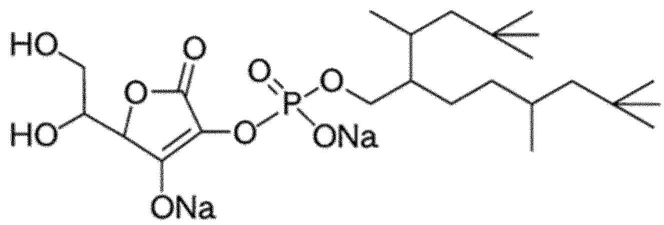

Examples of the phospholipid used in the present invention include, e.g., a natural phospholipid such as egg yolk lecithin or soybean lecithin; a phospholipid such as hydrogenated egg yolk lecithin or soybean lecithin in which an unsaturated carbon chain is converted into a saturated bond form by hydrogenation; and phosphatidylcholine, phosphatidylethanolamine, phosphatidylserine, phosphatidic acid, phosphatidylinositol, and phosphatidylglycerol that are purified from natural lecithin or synthesized.

Such a phospholipid can be used alone, or two or more of such phospholipids can be used in combination. Hydrogenated lecithin or hydrogenated lecithin having an increased concentration of a specific phospholipid may be used.

As the phospholipid used in the present invention, a phospholipid obtained by purifying natural lecithin and adjusting the content of phosphatidylcholine to 55 to 85% by mass is stable, and preferable for preparing a fine capsule preparation. If the phospholipid includes an unsaturated bond, a hydrogenation treatment improves the stability of the fine-particulate capsule preparation.

Such phospholipids are well known, and commercially available products can be used such as Lecinol S-10 M and Lecinol S-10 E made by Nikko Chemicals Co., Ltd., Basis LP-60 HR made by Nisshin OilliO Group, Ltd., COATSOMENC-61 made by NOF CORPORATION, and egg yolk lecithin PL 100 P made by Kewpie Corporation. In addition to the above, examples of commercially available products that can be used include, e.g., Lecinol S-10 and Lecinol S-10 EX made by Nikko Chemicals Co., Ltd., Basis LP-60, Basis LP-20, and Basis LP-20 H made by Nisshin OilliO Group, Ltd., COATSOMENC-21 and COATSOMENC-20 made by NOF CORPORATION, and egg yolk lecithin PL-60, egg yolk lecithin PL- 100H, egg yolk lecithin PL-30, egg yolk lecithin PL95P, and egg yolk lecithin PL98P made by Kewpie Corporation.

The blending amount of a phospholipid (or phospholipids) added when preparing the topical fine-particulate capsule preparation of the present invention is preferably 0.1 to 10% by mass. In order to prepare a stable capsule preparation, the blending amount is more preferably 1.0 to 2.0% by mass.

The ceramide analog used in the present invention contains a ceramide or a similar ingredient. Such a ceramide analog may be derived from, e.g., a synthetic product or an extracted product, and, at least one selected from a natural ceramide, a glycosylated ceramide compound, a synthetic pseudo ceramide (ceramide analog), sphingosine, and phytosphingosine can be used.

Natural ceramides are well-known compounds also referred to as ceramide 1, ceramide 2, ceramide 3, ceramide 4, ceramide 5, ceramide 6, ceramide 7, ceramide 8, and ceramide 9, and commercially available ones can be used depending on the purpose.

These ceramides are natural products derived from humans, animals, and the like, and there are various modifications with respect to the length of the alkyl chain. With respect to the length of the alkyl chain, the ceramides may have any structure as long as they have the above skeleton.

In addition, in order to impart solubility for the purpose of formulation or the like, it is also possible to use the ceramides to which modification has been added depending on the purpose, for example, a double bond is introduced into the molecule or a hydrophobic group is introduced for imparting permeability. Either the D(-) form or the L(+) form of optically active substances of these ceramides may be used, or a mixture thereof may be used.

Examples of commercially available ceramides include, e.g., Ceramide I, Ceramide III, Ceramide IIIB, and Ceramide VI made by Evonik Operations GmbH, CERAMIDE 2 made by Croda Japan KK, Ceramide TIC-001 made by TAKASAGO INTERNATIONAL CORPORATION, and DS-ceramide Y30 and DS-ceramide Y3S made by Doosan Corporation.

Another example of the ceramide analog in the present invention includes a glycosylated ceramide compound containing saccharide in the molecule.

Examples of the saccharide used for modification of the ceramide include, e.g., monosaccharides such as glucose and galactose, disaccharides such as lactose and maltose, and oligosaccharides and polysaccharides obtained by polymerizing such monosaccharides and/or disaccharides with a glucoside bond. A saccharide derivative may be used in which a hydroxyl group in a saccharide unit is replaced with another group. Examples of such a saccharide derivative include, e.g., glucosamine, glucuronic acid, and N-acetylglucosamine. Among them, from the viewpoint of dispersion stability, saccharides having 1 to 5 saccharide units are preferable, specifically, glucose and lactose are preferable, and glucose is more preferable.

The saccharide ceramide compound can be obtained by synthesis or as a commercially available product. Examples thereof include, e.g., Biocera G, Biocera QD, and Delisome CX2 made by Dainihonkasei Co., Ltd., and Biosphingo made by Kikkoman Biochemifa Company.

In addition, still another example of the ceramide analog in the present invention includes synthetic pseudo ceramides (also referred to as synthetic similar ceramides or synthetic ceramide analogs. For example, N-(hexadecyloxyhydroxypropyl)-N-hydroxyethyl hexadecanamide or the like) that mimic the structure of ceramides.

Furthermore, other examples of the ceramide analog in the present invention include sphingosine and phytosphingosine. These may be synthetic or natural products, and natural sphingosine and sphingosine analogs can be used. Such compounds are also included in the ceramide analog of the present invention.

Specific examples of the natural sphingosine include sphingosine, dihydrosphingosine, phytosphingosine, sphingadienine, dehydrosphingosine, dehydrophytosphingosine, and N-alkyl forms (for example, N-methyl forms) and acetylated forms thereof.

Either the D(-) form or the L(+) form of optically active substances of the sphingosine may be used, or a mixture of a natural form and a non-natural form may be used.

The relative configuration of the compound may be a natural configuration, a non-natural configuration, or a mixture thereof. Among them, specific examples of the phytosphingosine that can be suitably used in the present invention includes, e.g., NIKKOL Ceralipid PS 236 made by Nikko Chemicals Co., Ltd., DS-PHYTOSPHINGOSINE made by Doosan Corporation, and Phytosphingosine made by Evonik Operations GmbH. As the phytosphingosine, either a naturally derived extract or a synthetic product may be used.

In the capsule preparation of the present invention, only one kind of the above-mentioned ceramide analogs may be used, or two or more kinds thereof may be used in combination.

The concentration of the ceramide analog when preparing the topical fine-particulate capsule preparation of the present invention is preferably 0.1 to 10% by mass, and particularly preferably 1 to 3% by mass for preparing a stable capsule preparation.

The polyglycerin fatty acid ester used in the present invention is an ester of a polyglycerin having an average degree of polymerization of 2 or more, preferably 6 to 15, and more preferably 8 to 10, and a fatty acid having 8 to 18 carbon atoms, for example, caprylic acid, capric acid, lauric acid, myristic acid, palmitic acid, stearic acid, oleic acid, or linoleic acid.

Preferred examples of the polyglycerin fatty acid ester include, e.g., hexaglycerin monooleate, hexaglycerin monostearate, hexaglycerin monopalmitate, hexaglycerin monomyristate, hexaglycerin monolaurate, decaglycerin monooleate, decaglycerin monostearate, decaglycerin monopalmitate, decaglycerin monomyristate, and decaglycerin monolaurate. These esters are well-known raw materials added as emulsifiers or foaming agents to pharmaceuticals, cosmetic products, foods, and the like, and commercially available products can be used. In the capsule preparation of the present invention, only one kind of such polyglycerin fatty acid esters may be used, or two or more kinds thereof may be used in combination.

The concentration of the polyglycerin fatty acid ester(s) when preparing the topical fine-particulate capsule preparation of the present invention is preferably 1 to 20% by mass. In order to prepare a stable capsule preparation, 5 to 20% by mass is particularly preferred.

A composition example of the topical fine-particulate capsule preparation of the present invention is now described. The capsule preparation can be prepared by (i) mixing and stirring, with an aqueous solvent such as an aqueous glycerin solution, 1 to 10% by mass of the isostearyl ascorbyl phosphate or its metal salt (disodium isostearyl ascorbyl phosphate), and 1 to 20% by mass of the phospholipid, the ceramide analog, and a fatty acid glyceryl derivative; (ii) adding thereto 1 to 30 wt% of an oily ingredient as necessary; and (iii) dispersing and emulsifying the mixture using a homogenizer.

The thus-obtained topical fine-particulate capsule preparation is normally a substantially spherical fine-particulate microcapsule preparation having micron-sized particle diameters, for example, particle diameters of about several pm (1 to 5 pm).

The topical fine-particulate capsule preparation of the present invention is not limited to a microcapsule preparation, and may be a nanocapsule preparation.

That is, the microcapsule preparation obtained as described above can be further pulverized by mechanical shearing force, impact force, turbulent flow, or the like using a well-known emulsifier such as a high-pressure emulsifier (high-pressure homogenizer) or a thin film swirling type high-speed mixer to produce a nano-sized fine-particulate capsule preparation having particle diameters of less than 1 pm.

The fine-particulate capsule preparation of the present invention has excellent skin permeability, and the ability to effectively release the encapsulated ingredients in the skin, and is useful as a delivery preparation of which the active ingredients can be delivered into the skin, as verified in evaluation tests of Examples described later.

The capsule preparation of the present invention can be used as a fine-particulate capsule preparation having transdermal absorbability with the active ingredients contained in a skin topical agent such as a cosmetic material or a pharmaceutical agent. Also, the capsule preparation can be added to a different skin topical agent such that the effect of the active ingredients is produced. Thus, the capsule preparation can be prepared for various skin topical agents.

If the capsule preparation of the present invention is added in a required ratio to form a skin topical agent such as a cosmetic material or a pharmaceutical agent, one kind or two or more kinds of fine-particulate capsule preparations may be added.

Incidentally, there is no problem in safety even if a component known to be safe is excessively added, and such a component can be added at a concentration at which a skin topical agent such as a cosmetic material or a pharmaceutical agent does not lose practicability due to skin irritation.

The skin topical agent of the present invention can appropriately contain, in addition to the above essential ingredients, various ingredients for use in normal cosmetics, quasi-drugs, pharmaceutical agents, and the like, for example, an oily ingredient, an emulsifier, a moisturizer, a thickener, a medicinal ingredient, a preservative, a pigment, a powder, a pH adjuster, an ultraviolet absorber, an antioxidant, a fragrance, and the like.

Specific examples of the oily ingredient include, e.g., liquid paraffin, petrolatum, microcrystalline wax, squalane, jojoba oil, beeswax, carnauba wax, lanolin, olive oil, coconut oil, higher alcohols, fatty acids, esters of higher alcohols such as isostearyl alcohol and fatty acids, and silicone oils.

Examples of the emulsifier include, e.g., nonionic surfactants such as polyoxyethylene alkyl ether, polyoxyethylene fatty acid ester, polyoxyethylene sorbitan fatty acid ester, sorbitan fatty acid ester, glycerin fatty acid ester, polyglycerin fatty acid ester, and polyoxyethylene hydrogenated castor oil; anionic surfactants such as sodium stearoyl lactate; amphoteric surfactants such as soybean phospholipid; and cationic surfactants such as alkyltrimethylammonium chloride.

Examples of the moisturizer include, e.g., glycerin, sorbitol, xylitol, maltitol, propylene glycol, polyethylene glycol, 1,3-butylene glycol, and 1,2-pentanediol.

Examples of the thickener include, e.g., carboxyvinyl polymers, (acrylates/alkyl acrylates) cross polymers, xanthan gum, methyl cellulose, polyvinyl pyrrolidone, gelatin, and clay minerals such as bentonite.

Examples of the active ingredients or other medicinal ingredients include, e.g., antiaging agents such as various vitamins and derivatives thereof, allantoin, glycyrrhizic acid and derivatives thereof, and various animal and plant extracts, moisturizers, hair restorers, hair growth agents, transdermal absorption accelerators, ultraviolet absorbers, cell activators, anti-inflammatory agents, skin lightening agents, and antiseptic and antifungal agents.

A skin topical agent containing the capsule preparation of the present invention is not particularly limited in terms of production conditions, except that the fine-particulate capsule preparation is added as an active ingredient, and it can be prepared by a well-known cosmetic production method, too.

In addition, the use of the fine-particulate capsule preparation of the present invention is not limited by domestic and foreign laws, social circumstances, or the like, and the capsule preparation can also be applied to quasi-drugs, topical pharmaceutical agents, and the like. The dosage forms of cosmetics can be selected depending on the purpose, and, for example, a cream form, an emulsion form, a liquid form, a gel form, an ointment form, a pack form, a stick form, a powder form, and the like can be used.

EXAMPLES

A topical fine-particulate capsule preparation according to each of the below-described Examples 1 to 18 and Comparative Examples 1 to 6 was prepared by (i) adding amphiphilic capsule membrane ingredients (A1), other capsule membrane ingredients (A2), encapsulated (oily) ingredients (B), and water or an aqueous ingredient (C) in the proportion shown in Table 1 or Table 2 (total 100 wt%) ; and (ii) dispersing and emulsifying the mixture.

With respect to the capsule preparation obtained according to each of the Examples and Comparative Examples, a particle diameter thereof was measured using a laser diffraction/scattering type particle diameter distribution measuring device (LA-950 made by HORIBA). The measurement results are also shown in Tables 1 and 2.

[Examples 1 to 3 and Comparative Example 1]

In Example 1, a capsule preparation having the particle diameter shown in Table 1 was obtained by (i) mixing together 0.5% by mass of the disodium isostearyl ascorbyl phosphate represented by the above Chemical Formula 2 (hereinafter simply referred to as "the disodium isostearyl ascorbyl phosphate "), 2.0% by mass of commercially available hydrogenated lecithin, 50.0% by mass of glycerin, and water; (ii) adding thereto, as encapsulated ingredients, 0.1% by mass of retinol, 10.0% by mass of isostearic acid, 5.0% by mass of cetyl ethylhexanoate, 10.0% by mass of olefin oligomer, and 0.2% by mass of behenyl alcohol; and (iii) dispersing and emulsifying the mixture with a homogenizer.

In each of Examples 2 and 3 and Comparative Example 1, too, by, as in Example 1, adding ingredients in the proportion shown in Table 1, a capsule preparation according to Example 2, 3 was obtained in which the amount of the disodium isostearyl ascorbyl phosphate added as a capsule membrane ingredient was changed. Also, a fine-particulate capsule preparation according to Comparative Example 1 was obtained which does not include the disodium isostearyl ascorbyl phosphate.

[Examples 4 to 6 and Comparative Example 2]

In Example 4, a capsule preparation was obtained by (i) mixing together 0.5% by mass of the disodium isostearyl ascorbyl phosphate, 2.5% by mass of sphingomonas extract commercially available as a ceramide analog, 45.0% by mass of glycerin, and water; (ii) adding thereto, as encapsulated ingredients, 0.1% by mass of tocopherol, 20.0% by mass of isostearic acid and 5.0% by mass of cetyl ethylhexanoate; and (iii) dispersing and emulsifying the mixture with a homogenizer.

In Example 5, a capsule preparation was obtained in which the amount of the disodium isostearyl ascorbyl phosphate added is different from Example 4. In Example 6, a capsule preparation was obtained which does not include sphingomonas extract and includes only the disodium isostearyl ascorbyl phosphate (as a capsule membrane ingredient). In Comparative Example 2, a capsule preparation was obtained which includes only sphingomonas extract (as a capsule membrane ingredient).

[Examples 7 to 9 and Comparative Example 3]

In Example 7, a capsule preparation was obtained by (i) mixing together 0.5% by mass of the disodium isostearyl ascorbyl phosphate, 5.0% by mass of commercially available polyglyceryl diisostearate, 5.0% by mass of decaglyceryl monomyristate, 40.0% by mass of glycerin, and water; (ii) adding thereto, as encapsulated ingredients, 0.1% by mass of tocopherol, 5.0% by mass of cetyl ethylhexanoate, 5.0% by mass of squalane, 10.0% by mass of olefin oligomer, and 0.3% by mass of behenyl alcohol; and (iii) dispersing and emulsifying the mixture with a homogenizer.

In Example 8, a capsule preparation was prepared in which the amount of the disodium isostearyl ascorbyl phosphate added is different from Example 7. In Example 9, a capsule preparation was prepared which includes only the disodium isostearyl ascorbyl phosphate (as a capsule membrane ingredient). In Comparative Example 3, a capsule preparation was prepared which includes only polyglyceryl diisostearate, and decaglyceryl monomyristate (as capsule membrane ingredients).

[Table 1]

| Example number / Proportion of ingredients added and particle diameter | Example | | | | | | | | | Comparative Example | | |
|---|---|---|---|---|---|---|---|---|---|---|---|---|
| | 1 | 2 | 3 | 4 | 5 | 6 | 7 | 8 | 9 | 1 | 2 | 3 |
| (A1) Disodium isostearyl ascorbyl phosphate | 0.5 | 1.0 | 2.0 | 0.5 | 1.0 | 2.0 | 0.5 | 1.0 | 2.0 | – | – | – |
| (A1) Hydrogenated lecithin | 2.0 | 2.0 | – | – | – | – | – | – | – | 2.0 | – | – |
| (A2) Sphingomonas extract | – | – | – | 2.5 | 2.5 | – | – | – | – | – | 2.5 | – |
| (A2) Polyglyceryl diisostearate | – | – | – | – | – | – | 5.0 | 5.0 | – | – | – | 5.0 |
| (A2) Decaglyceryl monomyristate | – | – | – | – | – | – | 5.0 | 5.0 | – | – | – | 5.0 |
| (B) Retinol | 0.1 | 0.1 | 0.1 | – | – | – | – | – | – | 0.1 | – | – |
| (B) Tocopherol | – | – | – | 0.1 | 0.1 | 0.1 | 0.1 | 0.1 | 0.1 | – | 0.1 | 0.1 |
| (B) Isostearic acid | 10.0 | 10.0 | 10.0 | 20.0 | 20.0 | 20.0 | – | – | – | 10.0 | 20.0 | – |
| (B) Cetyl ethylhexanoate | 5.0 | 5.0 | 5.0 | 5.0 | 5.0 | 5.0 | 5.0 | 5.0 | 5.0 | 5.0 | 5.0 | 5.0 |
| (B) Squalane | – | – | – | – | – | – | 5.0 | 5.0 | 5.0 | – | – | 5.0 |
| (B) Olefin oligomer | 10.0 | 10.0 | 10.0 | – | – | – | 10.0 | 10.0 | 10.0 | 10.0 | – | 10.0 |
| (B) Behenyl alcohol | 0.2 | 0.2 | 0.2 | – | – | – | 0.3 | 0.3 | 0.3 | 0.2 | – | 0.3 |
| (C) Glycerin | 50.0 | 50.0 | 50.0 | 45.0 | 45.0 | 45.0 | 40.0 | 40.0 | 40.0 | 50.0 | 45.0 | 40.0 |
| (C) Purified water | Balance | Balance | Balance | Balance | Balance | Balance | Balance | Balance | Balance | Balance | Balance | Balance |
| Particle Diameter (μm) | 1.52 | 1.23 | 1.38 | 1.45 | 1.19 | 1.32 | 1.59 | 1.27 | 1.24 | 1.59 | 1.62 | 1.65 |

[Examples 10 to 18 and Comparative Examples 4 to 6]

In each of Examples 10 to 18 and Comparative Examples 4 to 6, by pulverizing, with a thin film swirling type high-speed mixer, a capsule preparation containing ingredients in the proportion shown in Table 2 below, a topical fine-particulate capsule preparation having the particle diameter shown in Table 2 was obtained.

[Table 2]

| Example number / Proportion of ingredients added and particle diameter | Example | | | | | | | | | Comparative Example | | |
|---|---|---|---|---|---|---|---|---|---|---|---|---|
| | 10 | 11 | 12 | 13 | 14 | 15 | 16 | 17 | 18 | 4 | 5 | 6 |
| (A1) Disodium isostearyl ascorbyl phosphate | 0.5 | 1.0 | 2.0 | 0.5 | 1.0 | 2.0 | 0.5 | 1.0 | 2.0 | – | – | – |
| (A1) Hydrogenated lecithin | 2.0 | 2.0 | – | – | – | – | – | – | – | 2.0 | – | – |
| (A2) Sphingomonas extract | – | – | – | 2.5 | 2.5 | – | – | – | – | – | 2.5 | – |
| (A2) Polyglyceryl diisostearate | – | – | – | – | – | – | 5.0 | 5.0 | – | – | – | 5.0 |
| (A2) Decaglyceryl monomyristate | – | – | – | – | – | – | 5.0 | 5.0 | – | – | – | 5.0 |
| (B) Retinol | 0.1 | 0.1 | 0.1 | – | – | – | – | – | – | 0.1 | – | – |
| (B) Tocopherol | – | – | – | 0.1 | 0.1 | 0.1 | 0.1 | 0.1 | 0.1 | – | 0.1 | 0.1 |
| (B) Isostearic acid | 10.0 | 10.0 | 10.0 | 20.0 | 20.0 | 20.0 | – | – | – | 10.0 | 20.0 | – |
| (B) Cetyl ethylhexanoate | 5.0 | 5.0 | 5.0 | 5.0 | 5.0 | 5.0 | 5.0 | 5.0 | 5.0 | 5.0 | 5.0 | 5.0 |
| (B) Squalane | – | – | – | – | – | – | 5.0 | 5.0 | 5.0 | – | – | 5.0 |
| (B) Olefin oligomer | 10.0 | 10.0 | 10.0 | – | – | – | 10.0 | 10.0 | 10.0 | 10.0 | – | 10.0 |
| (B) Behenyl alcohol | 0.2 | 0.2 | 0.2 | – | – | – | 0.3 | 0.3 | 0.3 | 0.2 | – | 0.3 |
| (C) Glycerin | 50.0 | 50.0 | 50.0 | 45.0 | 45.0 | 45.0 | 40.0 | 40.0 | 40.0 | 50.0 | 45.0 | 40.0 |
| (C) Purified water | Balance | Balance | Balance | Balance | Balance | Balance | Balance | Balance | Balance | Balance | Balance | Balance |
| Particle Diameter (nm) | 259 | 218 | 136 | 228 | 153 | 115 | 282 | 236 | 158 | 277 | 248 | 298 |

For each of the thus-obtained capsule preparations according to Examples 1 to 18 and Comparative Examples 1 to 6, a transdermal absorbability test was conducted as described below to check the transdermal absorbability and the supply properties of the encapsulated ingredients into the skin. The results are shown in Figs. 1 to 12.

<Transdermal absorbability test>

For the capsule preparation according to each of Example 1 to 18 and Comparative Example 1 to 6, a transdermal absorbability test was conducted using EPI-200X of Kurabo Industries Ltd., which is a three-dimensional cultured skin model composed of human normal epidermal keratinocytes.

That is, the above three-dimensional cultured skin model was totally cultured in a 6-well microplate using a dedicated medium. Then, 50 μL of a 50% aqueous solution of the capsule preparation was added to the upper portion of the skin model, and, after 24 hours passed, the culture solution under the skin model and the skin model were collected. By crushing, for extraction, the collected skin model using a homogenizer with 1 mL of a 50% ethanol aqueous solution, a skin model extract liquid was obtained. The encapsulated ingredients in the thus-collected culture solution and skin model extract liquid were quantified using a high-performance liquid chromatography (HPLC).

The total transdermal permeation amount was defined as the total amount of the encapsulated ingredients in the culture solution and the skin model extract liquid. The results are shown in Figs. 1 to 6.

In addition, the delivery rate (%) of the encapsulated ingredients in the skin was calculated using Calculation Formula A below, and whether the encapsulated ingredients were efficiently transported (delivered) to the target skin cell was evaluated. The results of the delivery rate (%) of the encapsulated ingredients in the skin are shown in Figs. 7 to 12.

(Calculation Formula A)

Delivery rate (%) of encapsulated ingredients in skin = (total amount of encapsulated ingredients in skin model extract liquid)/(total amount of encapsulated ingredients in culture solution and skin model extract liquid)

<Test Results>

As is apparent from the results shown in Figs. 1 to 6, too, for each of the microcapsule preparations of Examples 1 to 9 containing, as a capsule shell ingredient, hydrogenated lecithin, sphingomonas extract or polyglycerin fatty acid ester, since the disodium isostearyl ascorbyl phosphate has been introduced, the total transdermal permeation amount increased and also the delivery rate of the encapsulated ingredients in the skin improved compared to Comparative Examples 1 to 3, in which the disodium isostearyl ascorbyl phosphate was not added.

Also, as is apparent from the results shown in Figs. 7 to 12, too, for each of the nanocapsule preparations of Examples 10 to 13 containing, as a capsule shell ingredient, hydrogenated lecithin, sphingomonas extract or polyglycerin fatty acid ester, since the disodium isostearyl ascorbyl phosphate has been introduced, the total transdermal permeation amount increased and also the delivery rate of the encapsulated ingredients in the skin improved compared to Comparative Examples 4 to 6, in which the disodium isostearyl ascorbyl phosphate was not added.

It is apparent from the above that for the topical fine-particulate capsule preparations of Examples 1 to 18, which contain, as a capsule shell ingredient or ingredients, only the disodium isostearyl ascorbyl phosphate, or the disodium isostearyl ascorbyl phosphate and at least one selected from a phospholipid, a ceramide analog and a polyglycerin fatty acid ester, the delivery properties of the encapsulated ingredients into the skin are excellent.

Representative formulation examples of a skin topical agent are shown below in which a predetermined capsule preparation is used as an active ingredient. The numerical value at the right end of each row refers to the added ratio (% by mass). The skin topical product of each formulation example contains a capsule preparation including a predetermined amount of ingredients that can show efficacy derived from encapsulated ingredients.

[Formulation example 1] (Skin lotion)

| | |
|---|---|
| Capsule preparation A (Example 1) | 2.0 |
| Glycerin | 10.0 |
| Ethanol | 5.0 |
| POE (60) hydrogenated castor oil | 0.3 |
| Perfume | q.s. |

(continued)

| | |
|---|---|
| Preservative | q.s. |
| Purified water | Balance |

[Formulation example 2] (Gel cream)

| | |
|---|---|
| Fine-particulate capsule preparation A (Example 14) | 10.0 |
| Glycerin | 5.0 |
| Ethanol | 5.0 |
| Sodium hydroxide | 0.5 |
| Carboxyvinyl polymer | 0.8 |
| Perfume | q.s. |
| Preservative | q.s. |
| Purified water | Balance |

[Formulation example 3] (Milky lotion)

| | |
|---|---|
| Capsule preparation B (Example 6) | 5.0 |
| 1,3-butylene glycol | 10.0 |
| Carboxyvinyl polymer | 0.3 |
| Squalane | 5.0 |
| Cetanol | 0.6 |
| L-arginine | 0.3 |
| Perfume | q.s. |
| Preservative | q.s. |
| Purified water | Balance |

[Formulation example 4] (Cream)

| | |
|---|---|
| Fine-particulate capsule preparation B (Example 16) | 20.0 |
| 1,3-butylene glycol | 10.0 |
| Carboxyvinyl polymer | 0.4 |
| Squalane | 5.0 |
| Cetanol | 3.0 |
| Beeswax | 3.0 |
| L-arginine | 0.3 |
| Perfume | q.s. |
| Preservative | q.s. |
| Purified water | Balance |

INDUSTRIAL APPLICABILITY

The topical fine-particulate capsule preparation of the present invention can be used for many purposes taking advantage of its transdermal absorbability. If the encapsulated active ingredients are, e.g., physiologically active ingredients for skin that are effective as cosmetic materials, the capsule preparation can be used as a cosmetic product such as a cosmetic cream. Also, if the encapsulated active ingredients are medicinal ingredients for a living body, the capsule preparation can be used as a topical medicinal product having transdermal absorbability, e.g., cream or jelly.

## Claims

1. A topical fine-particulate capsule preparation comprising a fine-particulate capsule preparation in which an amphiphilic capsule membrane of emulsified particles encapsulates an active ingredient, wherein the amphiphilic capsule membrane is composed of an amphiphilic ingredient containing isostearyl ascorbyl phosphate or a metal salt thereof represented by the following Chemical Formula 1.

[Chemical Formula 1]

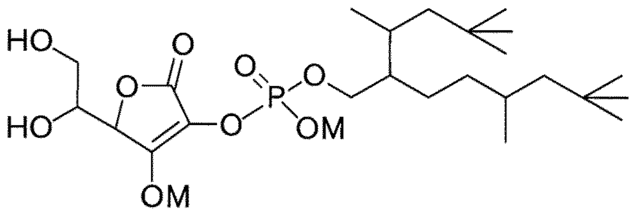

(M in Chemical Formula 1 is a hydrogen atom or a monovalent metal ion.)

2. The topical fine-particulate capsule preparation according to claim 1, wherein the amphiphilic capsule membrane contains a phospholipid.

3. The topical fine-particulate capsule preparation according to claim 1 or 2, wherein the amphiphilic capsule membrane contains one or more ceramide analogs selected from a natural ceramide, a glycosylated ceramide compound, a synthetic pseudo ceramide, sphingosine, and phytosphingosine.

4. The topical fine-particulate capsule preparation according to any one of claims 1 to 3, wherein the amphiphilic capsule membrane contains a polyglycerin fatty acid ester.

5. The topical fine-particulate capsule preparation according to any one of claims 1 to 4, wherein the emulsified particles have a particle diameter of 100 to 300 nm.

6. A skin topical agent containing the topical fine-particulate capsule preparation according to any one of claims 1 to 5, wherein the active ingredient is an active ingredient for skin physiological activity.

FIG. 1

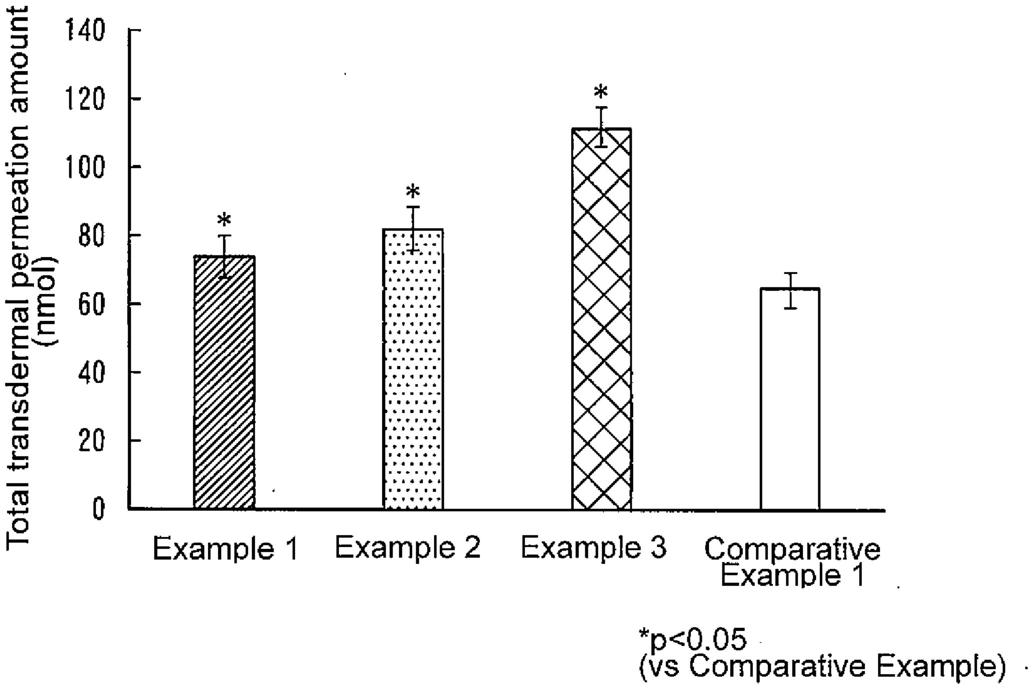
Total transdermal permeation amount (nmol)
140
120
100
80
60
40
20
0
*
*
*
Example 1
Example 2
Example 3
Comparative Example 1
*p<0.05
(vs Comparative Example)

FIG. 2

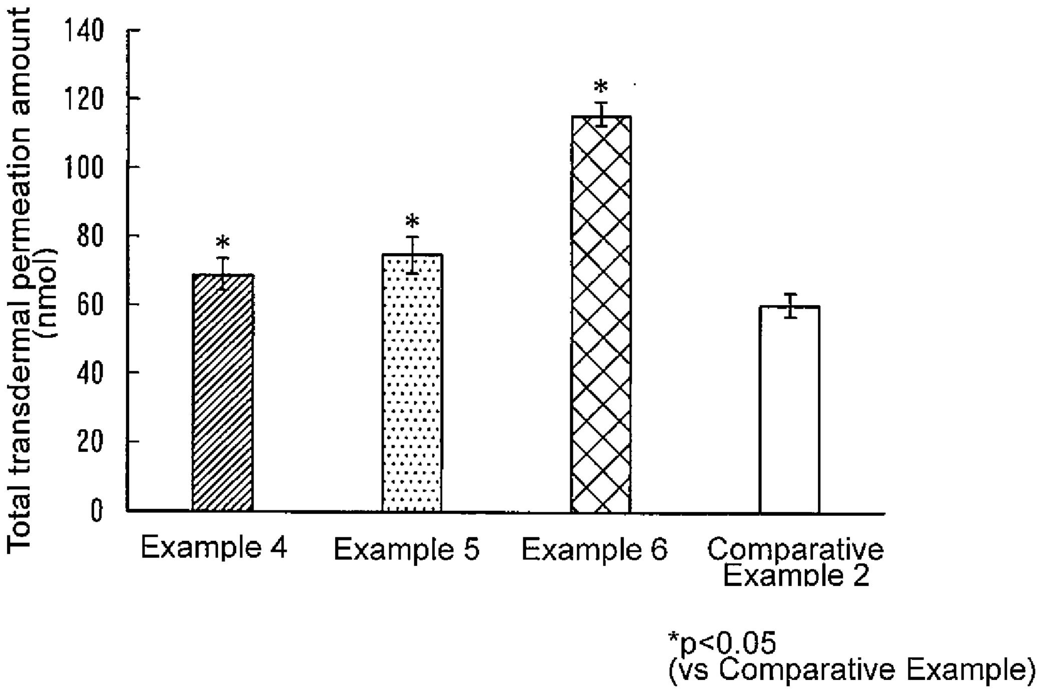
Total transdermal permeation amount (nmol)
140
120
100
80
60
40
20
0
*
*
*
Example 4
Example 5
Example 6
Comparative Example 2
*p<0.05
(vs Comparative Example)

FIG. 3

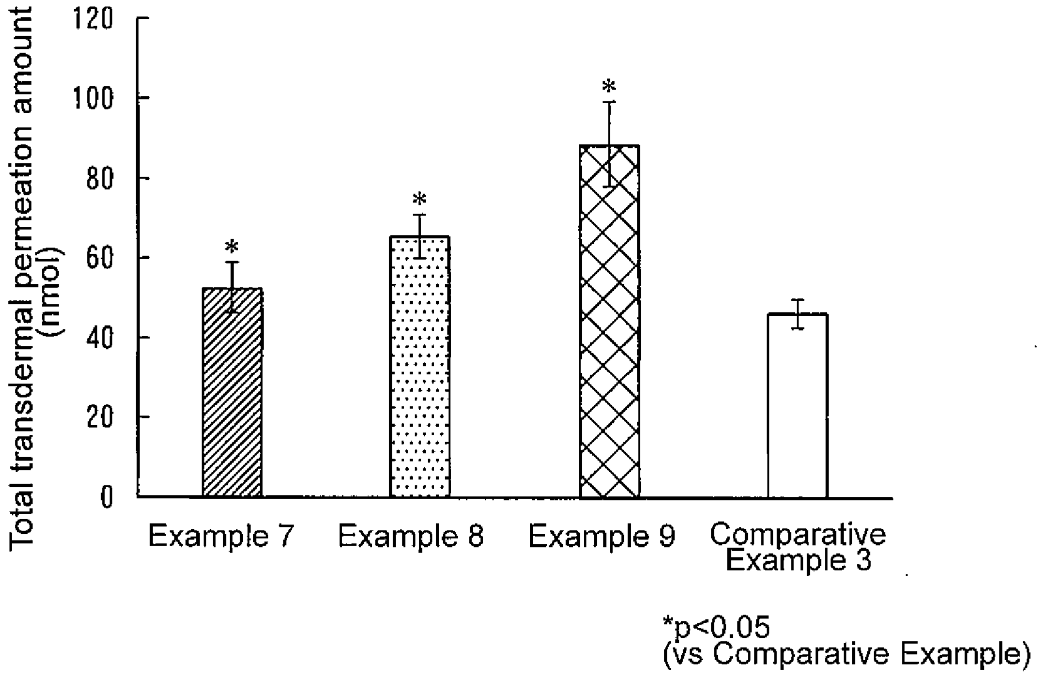
Total transdermal permeation amount (nmol)
120
100
80
60
40
20
0
Example 7
Example 8
Example 9
Comparative Example 3
*
*p<0.05
(vs Comparative Example)

FIG. 4

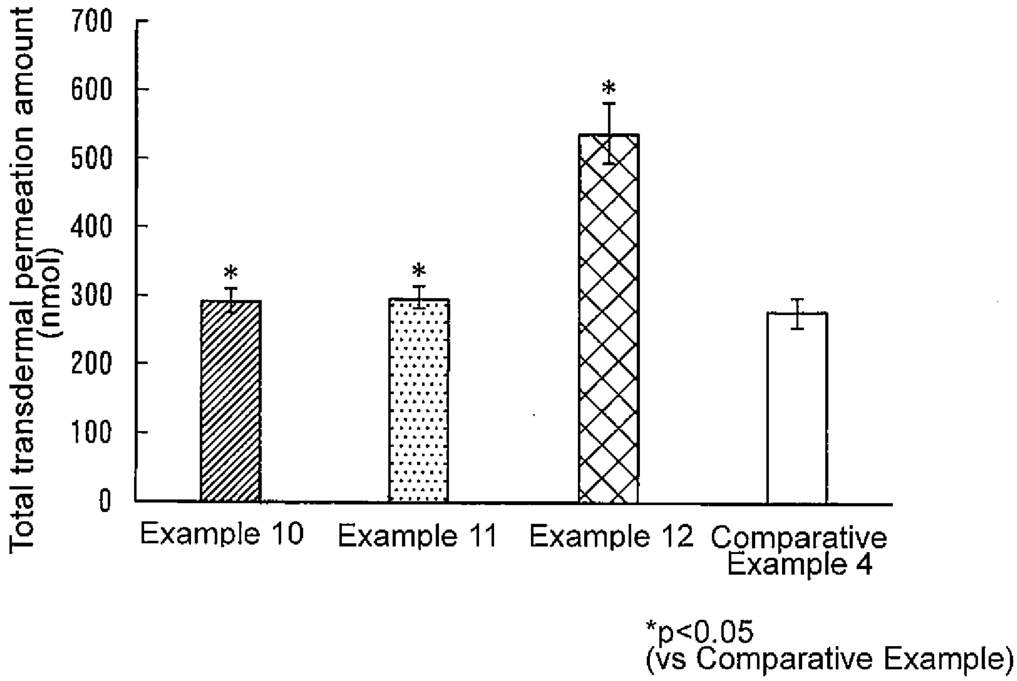
Total transdermal permeation amount (nmol)
700
600
500
400
300
200
100
0
Example 10
Example 11
Example 12
Comparative Example 4
*
*p<0.05
(vs Comparative Example)

FIG. 5

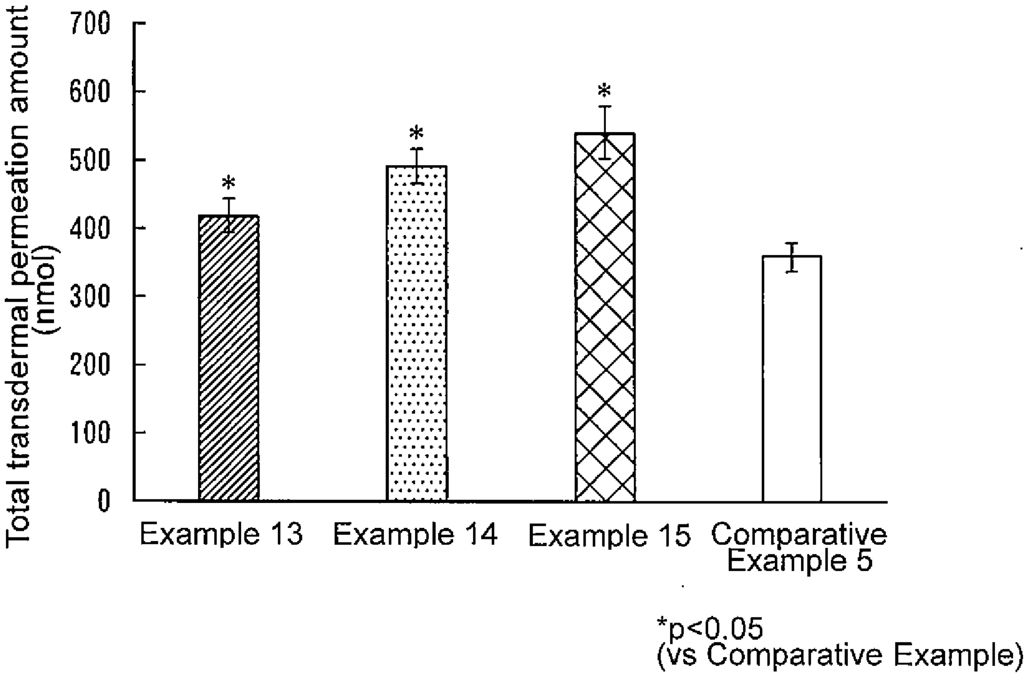
Total transdermal permeation amount (nmol)
700
600
500
400
300
200
100
0
*
*
*
Example 13
Example 14
Example 15
Comparative Example 5
*p<0.05
(vs Comparative Example)

FIG. 6

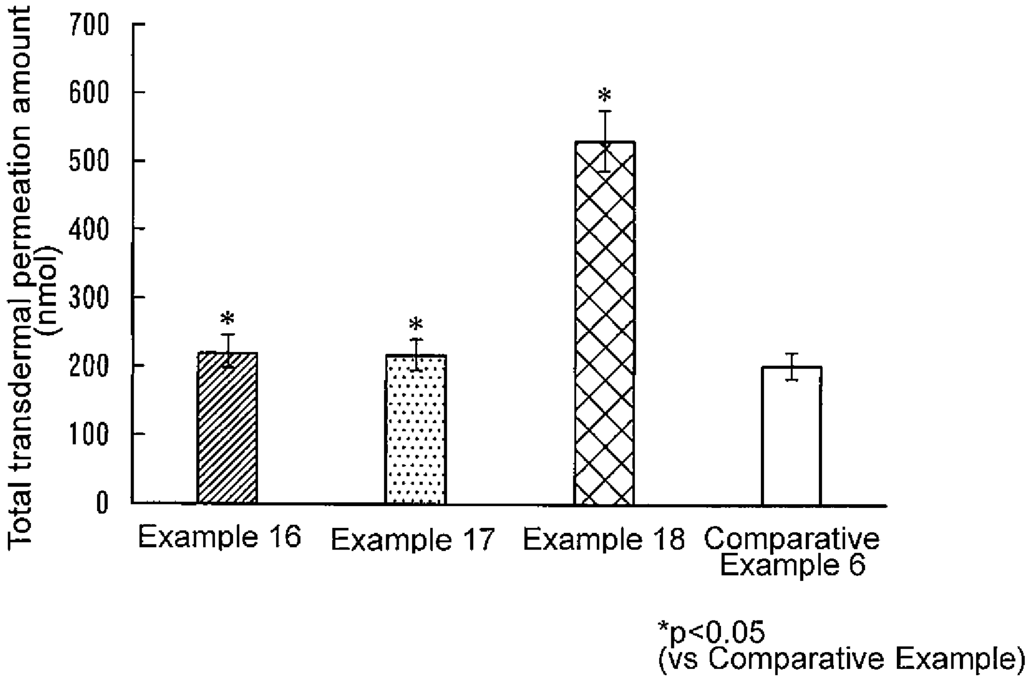
Total transdermal permeation amount (nmol)
700
600
500
400
300
200
100
0
*
*
*
Example 16
Example 17
Example 18
Comparative Example 6
*p<0.05
(vs Comparative Example)

FIG. 7

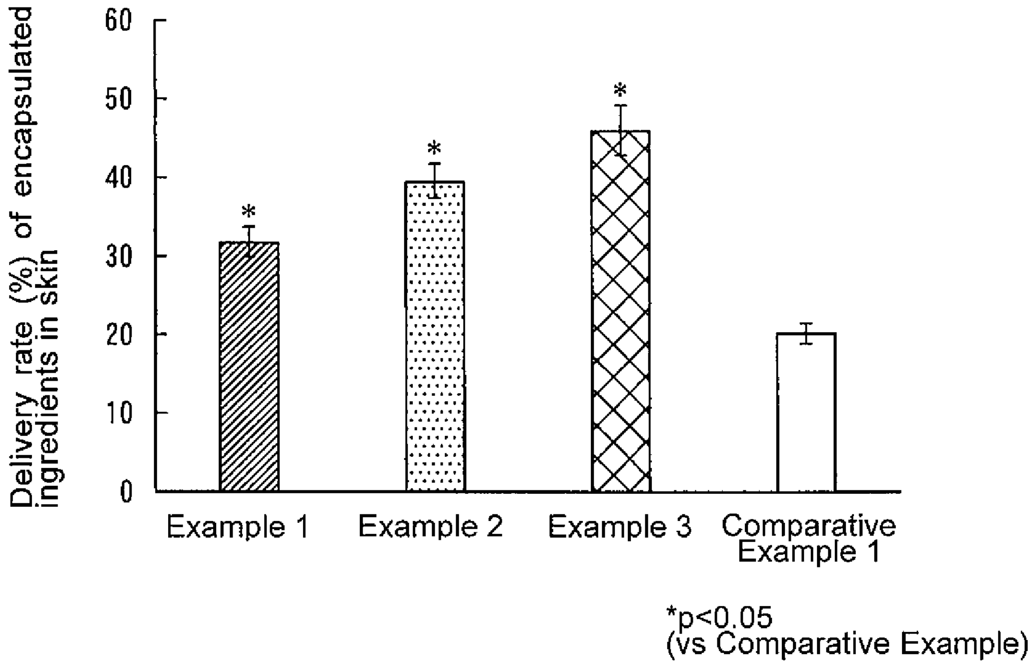
Delivery rate (%) of encapsulated ingredients in skin
60
50
40
30
20
10
0
*
Example 1
Example 2
Example 3
Comparative Example 1
*p<0.05
(vs Comparative Example)

FIG. 8

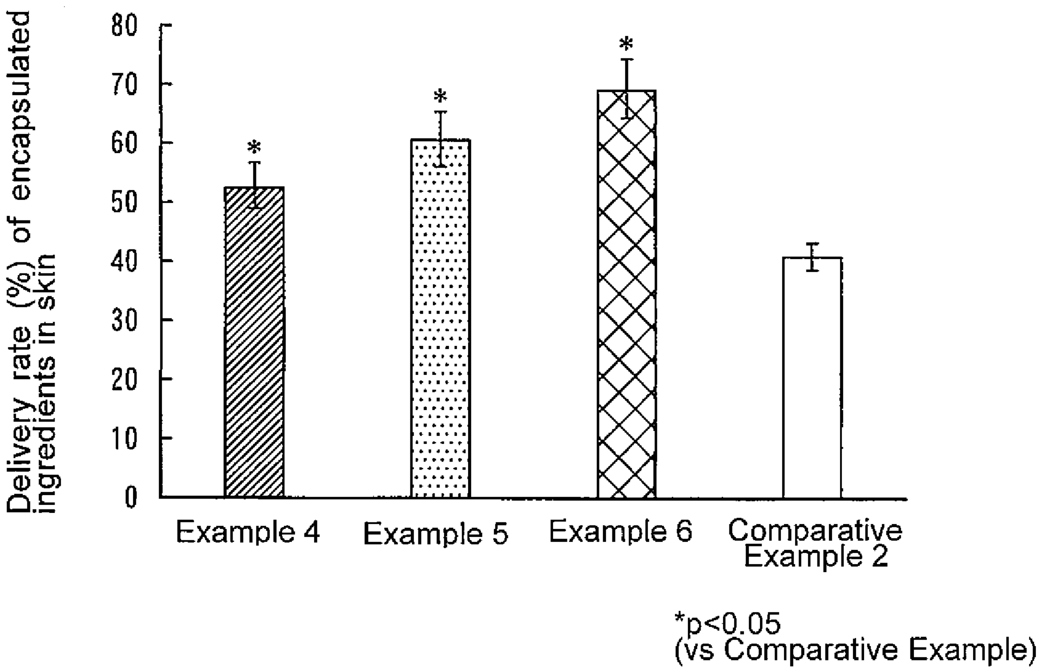
Delivery rate (%) of encapsulated ingredients in skin
80
70
60
50
40
30
20
10
0
*
Example 4
Example 5
Example 6
Comparative Example 2
*p<0.05
(vs Comparative Example)

FIG. 9

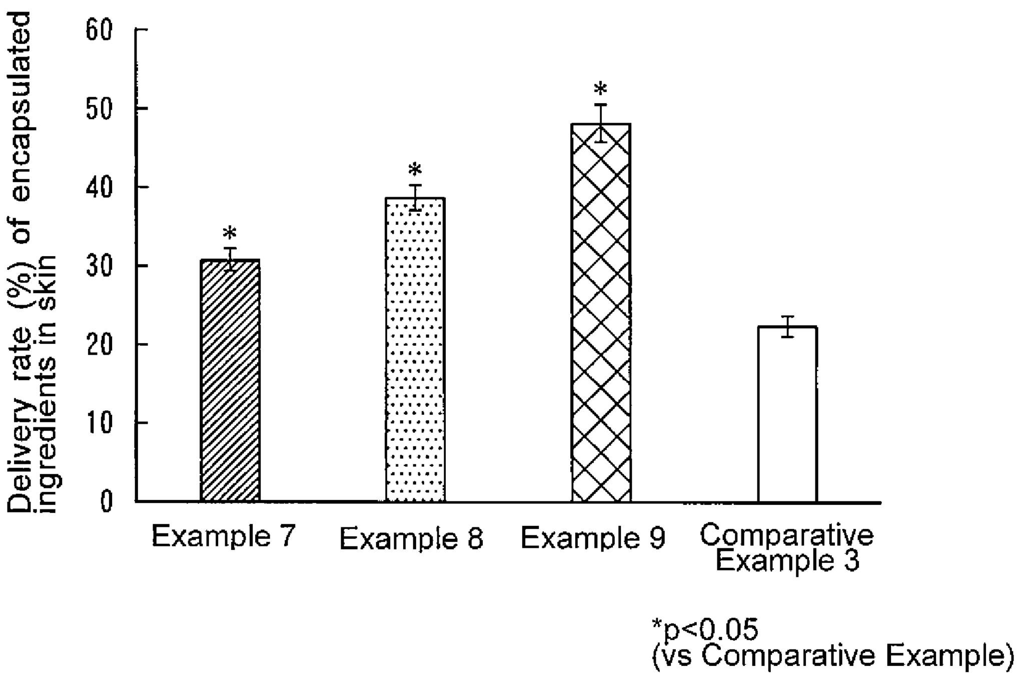
Delivery rate (%) of encapsulated ingredients in skin
60
50
40
30
20
10
0
*
Example 7
Example 8
Example 9
Comparative Example 3
*p<0.05
(vs Comparative Example)

FIG. 10

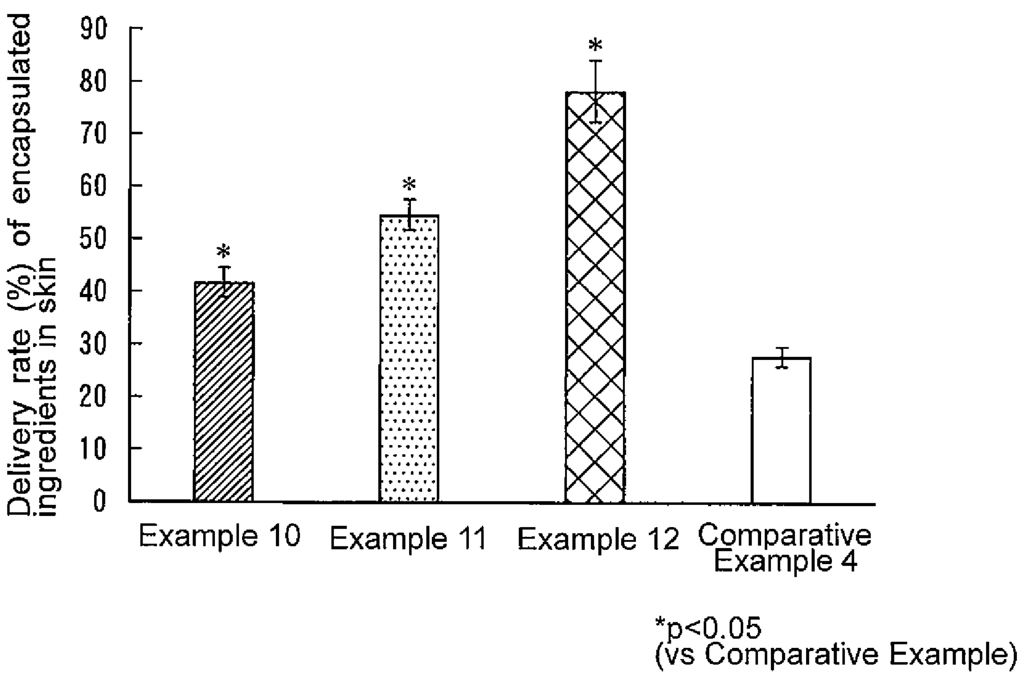
Delivery rate (%) of encapsulated ingredients in skin
90
80
70
60
50
40
30
20
10
0
*
Example 10
Example 11
Example 12
Comparative Example 4
*p<0.05
(vs Comparative Example)

FIG. 11

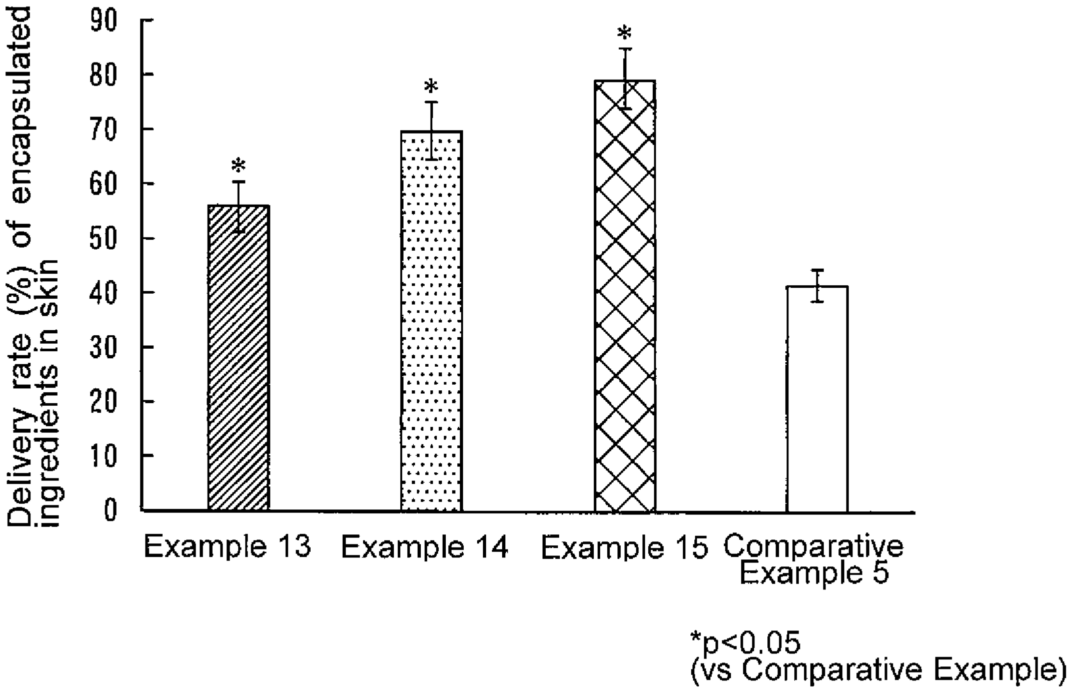
Delivery rate (%) of encapsulated ingredients in skin
90
80
70
60
50
40
30
20
10
0
*
Example 13
Example 14
Example 15
Comparative Example 5
*p<0.05
(vs Comparative Example)

FIG. 12

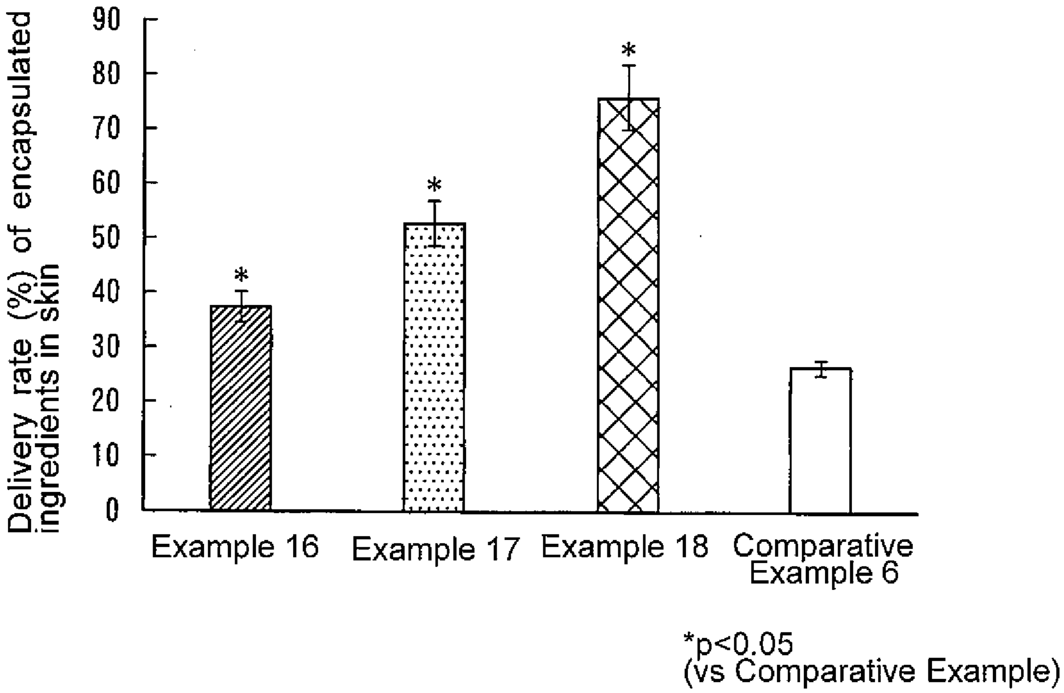
Delivery rate (%) of encapsulated ingredients in skin
90
80
70
60
50
40
30
20
10
0
*
Example 16
Example 17
Example 18
Comparative Example 6
*p<0.05
(vs Comparative Example)

## INTERNATIONAL SEARCH REPORT

International application No.
**PCT/JP2022/009187**

**A. CLASSIFICATION OF SUBJECT MATTER**

***A61K 47/24***(2006.01)i; ***A61K 8/11***(2006.01)i; ***A61K 9/127***(2006.01)i; ***A61K 47/14***(2006.01)i; ***A61P 17/00***(2006.01)i; ***A61Q 19/00***(2006.01)i
FI: A61K47/24; A61K9/127; A61K47/14; A61K8/11; A61Q19/00; A61P17/00

According to International Patent Classification (IPC) or to both national classification and IPC

**B. FIELDS SEARCHED**

Minimum documentation searched (classification system followed by classification symbols)

A61K47/24; A61K8/11; A61K9/127; A61K47/14; A61P17/00; A61Q19/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS/KOSMET (STN); Mintel GNPD

**C. DOCUMENTS CONSIDERED TO BE RELEVANT**

| Category* | Citation of document, with indication, where appropriate, of the relevant passages | Relevant to claim No. |
|---|---|---|
| X | Beauty Opener Gel III. ID 4973211, July 2017, Mintel GNPD [online], [retrieved on 06 January 2022], Internet <https://www.portal.mintel.com> page 4, column of "product description", pages 6-7, column of "product analysis", column of "ingredient" | 1-3, 6 |
| Y | | 1-6 |
| X | JP 2009-7299 A (TOYO BEAUTY KK) 15 January 2009 (2009-01-15) examples 3, 4, claims | 1-2, 4, 6 |
| Y | | 1-6 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 April 2022** | **17 May 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/009187**

**C. DOCUMENTS CONSIDERED TO BE RELEVANT**

| Category* | Citation of document, with indication, where appropriate, of the relevant passages | Relevant to claim No. |
|---|---|---|
| X<br><br>Y | IKEDA, M. et al. Protection of a novel nano-capsule with hydrolyzed egg shell membrane and an amphiphilic vitamin C derivative, disodium isostearyl 2-O-L-ascorbyl phosphate (VCP-IS-2NA), against UVA irradiation-induced Skin Damage on type III collagen reconstruction. 31st IFSCC Congress Yokohama 2020. 21-30 October 2020, Poster-241, October 2020, pages 2880-2885, Database Kosmet [Online] (retrieved on 2022 January 06): Retrieved from STN, Accession No.98627<br>column of "abstract" | 1, 6<br><br>1-6 |
| X<br><br>Y | 久間將義 ほか, 両親媒性ビタミンC誘導体を用いた抗酸化作用を有するナノカプセルにおける光老化に対する改善効果の検討, IFSCC2016オーランド大会・国内堯告会講演要旨集, 2016, page 43, (HISAMA, Masayoshi et al. Anti-photoaging of a Novel Nano-capsule with Amphiphilic Ascorbic Derivative due to Its Antioxidative Effect.), non-official translation (IFSCC2016 Orlando Convention/Domestic Report Meeting Abstracts.)<br>columns of "2. Method", "3. Results and Observations" | 1, 6<br><br>1-6 |
| X<br><br>Y | 久間將義ほか, 両親媒性ビタミンC誘導体を用いたナノカプセルにおける皮膚老化に対する改善作用, 日本薬学会年会要旨集, 第137年会, 2017, pages 158, 26PB-pm063, (HISAMA, Masayoshi et al.), non-official translation (Improvement effect on skin aging in nanocapsules using amphipathic vitamin C derivatives. Proceedings of The 137th Annual Meeting of the Pharmaceutical Society of Japan.)<br>columns of "Method", "Results and Observations" | 1, 6<br><br>1-6 |
| Y | JP 2011-219427 A (KYUSHU INST. OF TECHNOLOGY) 04 November 2011 (2011-11-04)<br>paragraph [0004] | 5 |
| Y | JP 2018-500359 A (CELLTRION, INC.) 11 January 2018 (2018-01-11)<br>paragraph [0019] | 5 |
| Y | JP 2013-180986 A (SAIENSURIN KK) 12 September 2013 (2013-09-12)<br>paragraph [0029] | 5 |
| Y | JP 2014-88360 A (ITO KK) 15 May 2014 (2014-05-15)<br>example 13 | 5 |

**INTERNATIONAL SEARCH REPORT**
**Information on patent family members**

International application No.
**PCT/JP2022/009187**

| Patent document cited in search report | Publication date (day/month/year) | Patent family member(s) | Publication date (day/month/year) |
|---|---|---|---|
| JP 2009-7299 A | 15 January 2009 | (Family: none) | |
| JP 2011-219427 A | 04 November 2011 | (Family: none) | |
| JP 2018-500359 A | 11 January 2018 | US 2018/0161254 A1<br>paragraph [0030]<br>EP 3241565 A2<br>KR 10-2016-0082483 A<br>CN 107106421 A | |
| JP 2013-180986 A | 12 September 2013 | (Family: none) | |
| JP 2014-88360 A | 15 May 2014 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2006306794 A
- JP 2010209012 A
- JP 2015209494 A

### Non-patent literature cited in the description

- *Skin Pharm. Physol.,* 2008, vol. 21, 235-248